# EUROPEAN PATENT SPECIFICATION

(11) **EP 1 575 974 B1**
(45) Date of publication and mention of the grant of the patent: **19.03.2008**
(21) Application number: 03775235.9
(22) Date of filing: 27.10.2003
(51) Int. Cl.: C07H 19/20, A23L 1/229

(54) **MODIFIED GUANOSINES HAVING FLAVOURING ACTIVITY**
MODIFIZIERTE GUANOSINE MIT GESCHMACKWIRKUNG
GUANOSINES MODIFIEES A ACTIVITE AROMATIQUE

(30) Priority: 30.10.2002 IT MI20022306
(43) Date of publication of application: 21.09.2005
(73) Proprietor: Fructamine S.P.A., 24030 Mozzo (IT)
(72) Inventor: FURIOSI, Carola, I-20134 Milano (IT); ZOIA, Andrea, I-20134 Milano (IT); GIZZONI, Claudio, I-20134 Milano (IT); COLOMBO, Enrico, I-20134 Milano (IT)
(74) Representative: Duffy, Assumpta Dympna
(86) International application number: PCT/EP2003/011913
(87) International publication number: WO 2004/039824

(56) References cited:
- US-A- 3 408 206
- SAKO, MAGOICHI ET AL: "A Convenient Method for the Preparation of N2-Ethylguanine Nucleosides and Nucleotides" JOURNAL OF ORGANIC CHEMISTRY (1999), 64(15), 5719-5721 , 1999, XP002270261
- NOONAN, TIMOTHY ET AL: "Interaction of GTP derivatives with cellular and oncogenic ras-p21 proteins" JOURNAL OF MEDICINAL CHEMISTRY (1991), 34(4), 1302-7 , 1991, XP002270262
- YAMAZAKI, SATOHIRO ET AL: "Synthesis of 2-alkylthionosine 5'-phosphates and N2-methylated guanosine 5'-phosphates" CHEMICAL & PHARMACEUTICAL BULLETIN (1968), 16(2), 338-44 , 1968, XP009025855 cited in the application

## Description

### FIELD OF THE INVENTION

The present invention relates to flavouring agents or flavour enhancers, in particular guanosine-5'-monophosphate (hereinafter also referred to as 5'-GMP) derivatives.

### BACKGROUND OF THE INVENTION

The term "Flavour enhancer" indicates substances which do not necessarily have a flavour of their own but, when added to foods or flavourings, improve their organoleptic characteristics, increasing their savoury flavour and giving an impression of richness, meatiness, continuity and mouthfeel. This property is associated with a flavour known as the"fifth flavour", called the "UMAMI flavour "by the Japanese.

Monosodium glutamate (hereinafter called"MSG") is widely used as a flavour enhancer; however, in a certain category of consumers it is responsible for a series of side effects commonly known as "Chinese restaurant syndrome".

These side effects can be partly reduced by associating 5'-monophosphate ribonucleotides, such as inosine 5'-monophosphate and guanosine 5'-monophosphate (hereinafter called IMP andGMP respectively), with MSG.

The use of modified ribonucleotides with superior activity to IMP and GMP would enable the quantity of MSG in foods and flavourings to be further reduced, thus reducing its side effects.

Yamazaki et al., Chem. Pharm. Bull., 1968, 16, 338-344 described the synthesis of 2-alkylthioinosine 5'-monophosphate and N²-methyl- and N², N²-dimethylguanosine 5'-monophosphate, while K. Imai, Chem.Pharm. Bull., 1971, 19, 576-586 described the synthesis of 2-thio- and 2-arylinosine 5'-monophosphate.

In Communications, 80, 1025-1028, 1980, Keemal and Breeze described a general method for the synthesis of N²-alkyl nucleosides and the preparation of N²-methyl-guanosine. It has now been discovered that a class of N²-alkyl guanosines possess advantageous flavouring properties which enable the dose, and therefore the side effects, of MSG in foods and flavourings to be limited.

Sako et al., J Org. Chem., 1999, 64, 5719-5721 discloses a method for the preparation of certain N²-ethylguanine nucleosides and nucleotides in a study of the molecular mechanisms explaining the carcinogenic effects of the primary metabolite of ethanol.

Noonan et al., J. Med. Chem., 1991, 34, 1302-1307 describes the synthesis of certain GTP derivatives and their interaction with cellular and oncogenic *ras-*p21 proteins.

US-A-3408206 discloses certain 5'-nucleotides which are capable of improving the taste of food.

### SUMMARY OF THE INVENTION

The present invention relates to the compounds of general formula(I) 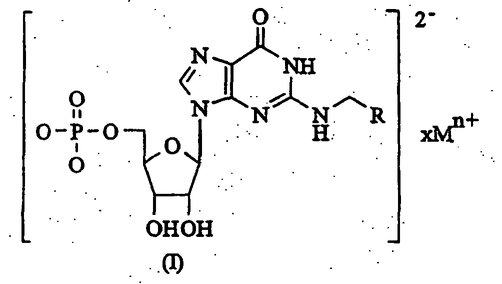 wherein R is:
- C₁-C₄ alkyl, optionally substituted with an S-R¹ group, in which R¹ is C₁-C₃ alkyl;
- a phenyl, benzyl, thiophenyl or benzothiophenyl group, optionally substituted with one or more -NO₂, -CHO, -R¹ or -SR¹ groups, in which R¹ has the meaning defined above;
- M is hydrogen, an alkali or alkaline-earth metal;
- X is 1 when n is 2 and X is 2 when n is 1;
with the exclusion of the compounds of formula (I) wherein R is unsubstituted methyl or propyl, M is hydrogen, X is 2 and n is 1.

The compounds of formula (I) are particularly useful as excipients in the preparation of foods in which 5'-monophosphate ribonucleotides are used together with monosodium glutamate.

The present invention also relates to the use of the compounds according to formula (I) as flavour enhancers and the use of a compound of formula (I) in admixture respectively with monosodium glutamate and/or 5'-monophosphate ribonucleotides.

### DETAILED DISCLOSURE OF THE INVENTION

The present invention provides the compounds of general formula (I) 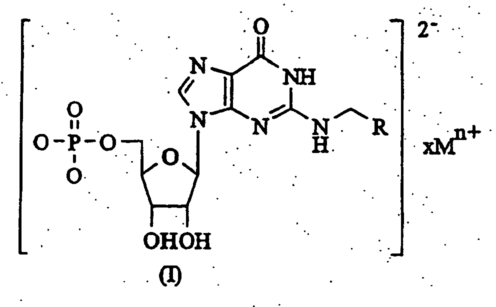 wherein R is:
- C₁-C₄ alkyl, optionally substituted with an S-R¹ group, in which R¹ is C₁-C₃ alkyl;
- a phenyl, benzyl, thiophenyl or benzothiophenyl group, optionally substituted with 1-5 substituents, preferably 1 to 3 substituents, which can be the same or different, selected from -NO₂, -CHO, -R¹ or -SR¹ groups, in which R¹ has the meaning defined above;
- M is hydrogen, an alkali or alkaline-earth metal;
   - X is 1 when n is 2 and X is 2 when n is 1;
      with the exclusion of the compounds of formula (I) wherein R is unsubstituted methyl or propyl, M is hydrogen, X is 2 and n is 1.

The C₁-C₄ alkyl group is selected from methyl, ethyl, propyl, isopropyl, n-butyl, sec-butyl, tert-butyl and preferably it is methyl or ethyl.

A first preferred group of compounds of formula (I) are those wherein R is a straight C₁-C₃ alkyl, optionally substituted with an S-R¹ group, in which R¹ is methyl, subject to the exclusion given above.

A second preferred group of compounds of formula (I) are those wherein R is a phenyl, thiophenyl or benzothiophenyl group, optionally substituted with an -NO₂, methyl or -SR¹ group, in which R¹ has the meaning defined above.

A third preferred group of compounds of formula (I) are those wherein M is hydrogen, sodium, potassium, calcium, magnesium or barium and preferably sodium.

A fourth preferred group of compounds of formula (I) are those in which M is hydrogen, sodium, potassium, calcium, magnesium or barium and R is:
- straight C₁-C₃ alkyl, optionally substituted with an -SR¹ group, in which R¹ corresponds to methyl, subject to the exclusion given above; or
- a phenyl, thiophenyl or benzothiophenyl group, optionally substituted with an -NO₂, methyl or -SR¹ group, in which R¹ has the meaning defined above.

Furthermore, particularly preferred are the following compounds:
N²-3-(methylthio)-propyl guanosine-5'-monophosphate ;
N²- (4-methylthiophenyl)-methyl guanosine-5'-monophosphate ;
N²-(2-methylthiophenyl)-methyl guanosine-5'-monophosphate;
N²-(thiophen-2-yl)-methyl guanosine-5'-monophosphate ;
N²-(thiophen-3-yl)-methyl guanosine-5'-monophosphate ;
N²- (5-methylthiophen-2-yl)-methyl guanosine-5'-monophosphate;
N²- (3-methylthiophen-2-yl)-methyl guanosine-5'-monophosphate;
N²- (5-ethylthiophen-2-yl)-methyl guanosine-5'-monophosphate;
N²-(5-nitrothiophen-2-yl)-methyl guanosine-5'-monophosphate ;
N²- (thianaphthen-3-yl)-methyl guanosine-5'-monophosphate
and the corresponding sodium salts.

Most preferred compounds are N²- (3-methylthio)-propyl guanosine-5'-monophosphate and the corresponding sodium salt.

The compounds of general formula (I) have flavour enhancers activity equal to or higher than that of 5'-GMP. The introduction of the indicated substituents on the purine base causes a 2 to 20 times increase in activity, usually 2 to 10 times, compared with that of 5'-IMP, commonly used in the reference organoleptic evaluations.

The present invention also provides the use of the compounds of general formula (I), alone or in combination, as flavouring agents for increasing the flavoury savour typical of the products containing monosodium glutamate and corresponding derivatives in mammals and preferably in humans.

The compounds of formula (I) described above can therefore be used to prepare pasta, risottos, soups, dried and wet soups, snacks, sauces, salad dressings, ready-made red and white sauces, stock cubes, soup preparations, cooked dressed meats. (ham, shoulder, reconstituted products, frankfurters, etc.) and uncooked dressed meats (salami, sausages, etc.), tinned meat, stuffed pasta and preserved vegetables, especially mushrooms.

The compounds of general formula (I) can be prepared with the process described in Kemal O., Communications, 1980, vol. 80,1025-1028 (scheme), which allows to prepare purine ribonucleosides selectively modified at the 2-position.

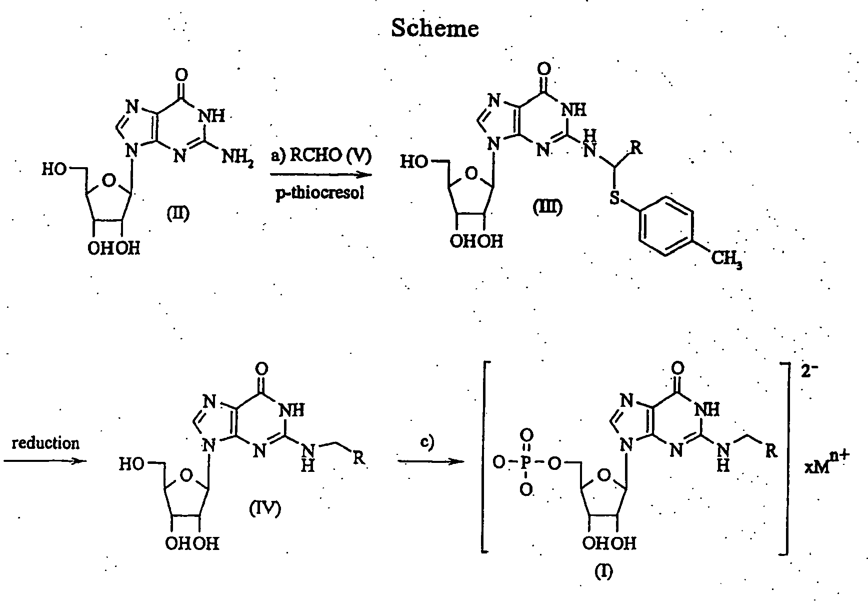

The process comprises reacting aldehydes R-CHO (V), in which R has the meanings defined above, and guanosine (II), in the presence of at least one mole of p-thiocresol, under acid catalytic conditions, preferably with acetic acid and preferably in aqueous medium, to yield the intermediate compounds of formula (III).

According to the present invention, preferred aldehydes are the following below indicated:
2-methylthiopropionaldehyde;
4-methylthiobenzaldehyde;
2-methylthiobenzaldehyde;
2-thiophenecarboxyaldehyde;
3-thiophenecarboxyaldehyde;
5-methyl-2-thiophenecarboxyaldehyde;
3-methyl-2-thiophenecarboxyaldehyde;
5-ethyl-2-thiophenecarboxyaldehyde;
5-nitro-2-thiophenecarboxyaldehyde;
thianaphthene-3-carboxaldehyde.

According to step a) of the Scheme, 2 to 40 mols of aldehyde of formula (V), 3 to 10 mols of p-thiocresol and an amount of acetic acid ranging from 2 to 15 mols are generally used.

The reaction is carried out preferably in aqueous, water-alcoholic or alcoholic solution, using a straight or branched C₁-C₄ alcohol, at a temperature usually ranging from 20 to 80°C and is completed in a time from 2 to 24 hours.

The compounds of formula (III) are usually recovered from the reaction mixture and purified by crystallization, using for example aqueous ethanol.

In the subsequent step b), the compound of formula (III) is reduced to give the corresponding derivative of formula (IV). The reduction is preferably carried out with sodium borohydride in an anhydrous solvent and under similar conditions to those described in literature. 1 to 3 mols of reducing agent per mole of (IV) are preferably used, at a temperature from 40 to 100°C; the reaction is usually completed in a time ranging from 2 to 4 hours.

Preferred solvents are 1,2-dimethoxyethane (glyme) and dimethylsulfoxide.

The phosphorylation reaction of N²-alkyl guanosine (IV), (step c), is carried out according to what described in K. Imai, J. Org. Chem., 1968, Vol. 34, No. 6, pp 1547-1550, using 1 to 4 mols of phosphorylating agent per mole of (IV) and at a temperature usually ranging from -10°C to 0°C. Phosphoryl chloride (POCl₃) is preferably used in the presence of trimethylphosphate. The reaction is completed in a time comprising from 6 to 24 hours and the compound of formula (I) is isolated from the mixture, for example by crystallization with aqueous ethanol.

### EXPERIMENTAL SECTION

### Organoleptic properties

The organoleptic properties of the compounds of formula (I) were evaluated in sensory tests by convening a panel of 10 tasters trained to recognise the umami flavour.

The derivatives were evaluated in aqueous solution at a neutral pH in synergy with monosodium glutamate (MSG), using as standard an aqueous solution at a neutral pH containing sodium inosinate (5'-IMP) and MSG, in which the concentration of MSG remained fixed and the concentration of 5'-IMP was varied, until the point of subjective equality with the solution of the derivative to be evaluated was reached.

The compounds according to the invention can be used as flavour enhancers in savoury products, in which monosodium glutamate (MSG) and ribonucleotides (5'-IMP and 5'-GMP) are normally used, either in synergy with the salt (NaCl) or alone.

The flavour-enhancing activity of the compounds of formula (I) proved to be 2 to 20 times greater than that of 5'-IMP in synergy with MSG (reference solution).

The threshold values of the compounds of formula (I) in aqueous solution with a neutral pH varied within a range of 0.02 to 0.006 g/100 ml.

The dosage of the compounds of formula (I) in the finished product, falls within the range of 5 to 2500 ppm, when they are used as flavour enhancers.

For example, a dose which arrive to 2500 ppm of compounds of formula (I) is used in the specific case of stock cubes, whereas the dose is generally between 5 and 100 ppm of the finished product in the case of use as a generic flavouring.

The flavour quality of the compounds of formula (I) has a variety of connotations. The flavours include meat, vegetable, mushroom, spices, tomato, boiled potato, etc..

A number of applications in which the organoleptic properties of the new sulphurised ribonucleotide derivatives have been evaluated in finished products are described below.

### Preparation 1: stock cube

The recipe (Table 1) uses a mixture of 5'-IMP and 5'-GMP, and the effect of replacing 5'-GMP with the new derivatives was evaluated.

**Table 1**

| INGREDIENTS | QUANTITY (g) |
|---|---|
| Salt | 50 |
| Monosodium glutamate (MSG) | 20 |
| Fats | 20 |
| Ribonucleotides (5'-GMP + 5'-IMP) | 0.5 |
| Meat extract | 2 |
| Vegetable extract - Yeast extract | 6.5 |
| Herbs and spices | 1 |
| | 100 |

The comparison was performed by taking the finished product according to the recipe set out in table 1 as reference.

The stock cube in the second formulation, containing guanosine sulphide derivatives, obtained scores 2 to 5 times higher than those of the reference formulation.

Other examples wherein the effect of the modified guanosine derivatives was evaluated are reported.

We chose commercial products which contain monosodium glutamate used as a flavour enhancer. We added a known quantity of compound of formula (I) (0.01 g) to these products, and performed the sensory evaluations by comparing the original product with the product to which the compound according to the invention was added.

### Preparation 2: Sachet of rice preparation

**Table 2**

| INGREDIENTS | QUANTITY (g) |
|---|---|
| Parboiled rice | 84.32 |
| Dried artichoke | 7 |
| Fats | 3 |
| MSG | 2 |
| Salt | 1.5 |
| Powdered skimmed milk | 1 |
| White onion powder | 0.5 |
| Flavourings | 0.5 |
| Herbs and spices | 0.18 |
| | 100 |

### Preparation 3: Meat-based filling for fresh pasta

**Table 3**

| INGREDIENTS | QUANTITY (g) |
|---|---|
| Pork and beef | 45 |
| Cheese | 20 |
| Breadcrumbs | 18 |
| Mortadella | 7 |
| Raw ham | 4 |
| Whey powder | 2 |
| Flavourings (*) | 1.5 |
| Salt | 1.5 |
| Parmesan cheese | 1 |
| | 100 |

| | |
|---|---|
| (*) Monosodium glutamate in filling 0.4 % (estimated) | |

In all cases, the products containing the compounds according to the invention obtained scores 2 to 5 times higher than those of the commercial product.

The example below illustrates the preparation of a compound of formula (I).

### N²-3'-methylthiopropyl guanosine-5'-monophosphate

### Step a): 2N-(p-tolylthio-3'-methylthiopropyl)-guanosine

In a 250 ml 2-necked round-bottom flask, equipped with condenser and thermometer, containing 45 ml of isopropanol, 3 g of guanosine (10.6 mmols), 4 g of p-thiocresol (32.2 mmols), 12 ml of methional (0.120 mols) and 6 ml of acetic acid are added under stirring and refluxed for 8-10 h.

After 2 h p-thiocresol (4 g) and 12 ml of methional are added again. The progress of the reaction is followed by TLC (eluent: ethyl acetate/ethanol/water = 100:20:10).

The mixture is allowed to cool and evaporated under reduced pressure. The liquid residue is washed with ether to obtain a gummy, pale yellow solid (4.7 g, 89.6%).

### Step b): N²-3'-methylthiopropyl guanosine

In a 25 ml 3-necked round-bottom flask, equipped with condenser, thermometer and plug, containing a solution of compound prepared as in step a) (4.7 g, 9.5 mmols) in 8 ml of DMSO, 470 mg (12.4 mmols) of sodium borohydride are added, under stirring and the mixture is heated to 100°C for 1.5 h, then cooled and neutralized with a 1 M solution of potassium dihydrogen phosphate. The resulting precipitate is filtered by suction and washed first with ice/water and then with sodium acetate.
3.5 g (94%) of a solid yellow product are obtained.

### Step c) N²-(3-methylthio)propyl guanosine-5'-monophosphate

2 ml of phosphoryl chloride are mixed with 13 ml of trimethyl phosphate and cooled to -10°C in a 3-necked round-bottom flask with calcium chloride guard-tube and thermometer. The solution is added, in small portions and under stirring, with 3.5 g (9.4 mmol) of compound prepared in step b), keeping the temperature below -5°C. After approx. 30 minutes the solution becomes transparent and viscous.

The mixture is stirred for approx. 3 hours keeping the temperature at -5°C. The reaction is quenched by pouring the solution in 600 ml of ice/water, then pH is adjusted to 2 with 4 N sodium hydroxide. The solution is percolated on a column (diameter = 3 cm) of animal activated charcoal (60 g), washed with distilled water to remove the salts and eluted with an ethanol/water/28% NH₄OH: 50/48/2 mixture.

The eluate is evaporated to dryness and the gummy residue is dissolved in 150 ml of water; pH is adjusted to 8.5 and to the solution is added under stirring 100 mg of barium acetate dissolved in some water.

The precipitate is centrifuged off and the resulting solution is added with 4 g of barium acetate in 30 ml of water. A further small portion of precipitate is obtained by addition of one volume of ethyl alcohol to the filtrate. The precipitate is isolated by centrifugation, then washed with ethanol, water and ether. The formed barium salt is suspended in 200 ml of water with 50 ml of Amberlite resin IR-120 (in Na+ form) and stirred until dissolution of the salt. The resin is filtered and the solution is reduced to 20 ml volume. Addition of ethyl alcohol yields an amorphous white precipitate (2.2 g, 51%).

## Claims

1. Compounds of general formula (I) 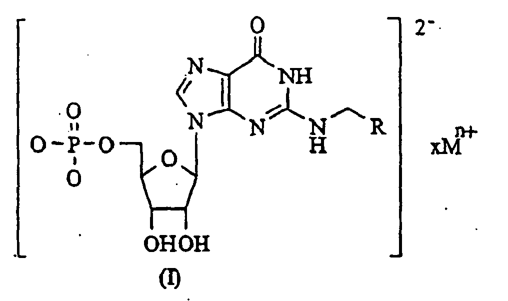 wherein R is:
- C₁-C₄ alkyl, optionally substituted with an S-R¹ group, in which R¹ is C₁-C₃ alkyl;
- a phenyl, benzyl, thiophenyl or benzothiophenyl group, optionally substituted with 1-5 substituents, preferably 1 to 3 substituents, which can be the same or different, selected from -NO₂, -CHO, -R¹ or -SR¹ groups, in which R¹ has the meaning defined above;
- M is hydrogen, an alkali or alkaline-earth metal;
- X is 1 when n is 2 and X is 2 when n is 1;
with the exclusion of the compounds of formula (I) wherein R is unsubstituted methyl or propyl, M is hydrogen, X is 2 and n is 1.

2. Compounds as claimed in claim 1 wherein R is straight C₁-C₃ alkyl, optionally substituted with an S-R¹ group, in which R¹ is methyl, subject to the exclusion in claim 1.

3. Compounds as claimed in claim 1, wherein R is a phenyl, thiophenyl or benzothiophenyl group, optionally substituted with an -NO₂, methyl or -SR¹ group, in which R¹ is as defined in claim 1.

4. Compounds as claimed in any one of claims 1 to 3, wherein M is hydrogen, sodium, potassium, calcium, magnesium or barium.

5. Compounds as claimed in claim 1, wherein M is hydrogen, sodium, potassium, calcium, magnesium or barium and R is:
- straight C₁-C₃ alkyl optionally substituted with a -SCH₃ group, subject to the exclusion in claim 1; or
- a phenyl, thiophenyl or benzothiophenyl group, optionally substituted with an -NO₂, methyl or -SCH₃ group.

6. Compounds as claimed in any one of claims 1 to 5 in which M is hydrogen or sodium.

7. A compound as claimed in claim 1 which is selected from:
N²- (3-methylthio)-propyl guanosine-5'-monophosphate;
N²- (4-methylthiophenyl)-methyl guanosine-5'-monophosphate;
N²- (2-methylthiophenyl)-methyl guanosine-5'-monophosphate;
N²-thiophen-2-yl-methyl guanosine-5'-monophosphate;
N²-thiophen-3-yl-methyl guanosine-5'-monophosphate;
N²- (5-methylthiophen-2-yl)-methyl guanosine-5'-monophosphate;
N²- (3-methylthiophen-2-yl)-methyl guanosine-5'-monophosphate;
N²- (3-ethylthiophen-2-yl)-methyl guanosine-5'-monophosphate;
N²- (3-nitrothiophen-2-yl)-methyl guanosine-5'-monophosphate;
N²-(thianaphthen-3-yl)-methyl guanosine-5'-monophosphate
and the corresponding sodium salts.

8. A compound as claimed in claim 7 which is N²-(3-methylthio)-propyl guanosine-5'-monophosphate and the corresponding sodium salt.

9. The use of the compounds as claimed in any one of claims 1 to 8 as flavour enhancers in food preparations.

10. The use of the compounds as claimed in any one of claims 1 to 8, alone or in combination with monosodium glutamate and/or with 5'-monophosphate nucleotides, as flavour enhancers in food preparations.

11. The use as claimed in claim 9 or 10 for the preparation of pasta, risottos, soups, dried and wet soups, snacks, sauces, salad dressings, ready-made red and white sauces, stock cubes, soup preparations, cooked and uncooked dressed meats, tinned meat, stuffed pasta and preserved vegetables.

12. Flavour enhancers comprising a compound of claims 1 to 8 in admixture with monosodium glutamate and/or with 5'-monophosphate nucleotides.

13. Food preparations containing the compositions of claim 12.

## Patentansprüche

1. Verbindungen der allgemeinen Formel (I) 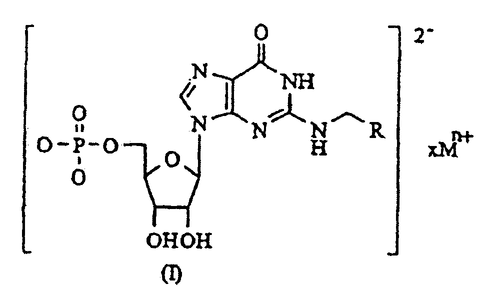 worin R für Folgendes steht:
- C₁-C₄-Alkyl, das gegebenenfalls mit einer S-R¹-Gruppe substituiert ist, in der R¹ für C₁-C₃-Alkyl steht;
- eine Phenyl-, Benzyl-, Thiophenyl- oder Benzothiophenyl-Gruppe, die gegebenenfalls mit 1-5 Substituenten substituiert ist, bevorzugt mit 1 bis 3 Substituenten, die die gleichen oder verschieden sein können, die aus -NO₂, -CHO, -R¹ oder -SR¹-Gruppen ausgewählt sind, in denen R¹ die vorstehend definierte Bedeutung hat;
- M ist Wasserstoff, ein Alkali- oder Erdalkalimetall;
- X ist 1, wenn n für 2 steht, und X ist 2, wenn n für 1 steht;
mit Ausschluss der Verbindungen der Formel (I), worin R für unsubstituiertes Methyl oder Propyl steht, M für Wasserstoff steht, X für 2 steht und n für 1 steht.

2. Verbindungen nach Anspruch 1, worin R für geradkettiges C₁-C₃-Alkyl steht, das gegebenenfalls mit einer S-R¹-Gruppe substituiert ist, in der R¹ für Methyl steht, unter Ausschluss in Anspruch 1.

3. Verbindungen nach Anspruch 1, worin R für eine Phenyl-, Thiophenyl- oder Benzothiophenyl-Gruppe steht, die gegebenenfalls mit einer -NO₂-, Methyl- oder -SR¹-Gruppe substituiert ist, in der R¹ wie in Anspruch 1 definiert ist.

4. Verbindungen nach einem der Ansprüche 1 bis 3, worin M für Wasserstoff, Natrium, Kalium, Calcium, Magnesium oder Barium steht.

5. Verbindungen nach Anspruch 1, worin M für Wasserstoff, Natrium, Kalium, Calcium, Magnesium oder Barium steht und R für Folgendes steht:
- geradkettiges C₁-C₃-Alkyl, das gegebenenfalls mit einer -SCH₃-Gruppe substituiert ist, unter Ausschluss in Anspruch 1; oder
- eine Phenyl-, Thiophenyl- oder Benzothiophenyl-Gruppe, die gegebenenfalls mit einer -NO₂-, Methyl- oder -SCH₃-Gruppe substituiert ist.

6. Verbindungen nach einem der Ansprüche 1 bis 5, in denen M für Wasserstoff oder Natrium steht.

7. Verbindung nach Anspruch 1, die aus Folgenden ausgewählt ist:
N²-(3-Methylthio)propylguanosin-5'-monophosphat;
N²-(4-Methylthiophenyl)methylguanosin-5'-monophosphat;
N²-(2-Methylthiophenyl)methylguanosin-5'-monophosphat;
N²-Thiophen-2-ylmethylguanosin-5'-monophosphat;
N²-Thiophen-3-ylmethylguanosin-5'-monophosphat;
N²-(5-Methylthiophen-2-yl)methylguanosin-5'-monophosphat;
N²-(3-Methylthiophen-2-yl)methylguanosin-5'-monophosphat;
N²-(3-Ethylthiophen-2-yl)methylguanosin-5'-monophosphat;
N²-(3-Nitrothiophen-2-yl)methylguanosin-5'-monophosphat;
N²-(Thianaphthen-3-yl)methylguanosin-5'-monophosphat
und den entsprechenden Natriumsalzen.

8. Verbindung nach Anspruch 7, die N²-(3-Methylthio)propylguanosin-5'-monophosphat und das entsprechende Natriumsalz ist.

9. Verwendung der Verbindungen nach einem der Ansprüche 1 bis 8 als Geschmacksverstärker in Nahrungsmittelzubereitungen.

10. Verwendung der Verbindungen nach einem der Ansprüche 1 bis 8, allein oder zusammen mit Mononatriumglutamat und/oder mit 5'-Monophosphatnukleotiden, als Geschmacksverstärker in Nahrungsmittelzubereitungen.

11. Verwendung nach Anspruch 9 oder 10 für die Zubereitung von Teigwaren, Risottos, Suppen, Trocken- und Nasssuppen, Imbissen, Soßen, Salatsoßen, gebrauchsfertigen roten und weißen Soßen, Brühwürfeln, Suppenzubereitungen, gekochtem und ungekochtem zubereitetem Fleisch, Dosenfleisch, gefüllten Teigwaren und konserviertem Gemüse.

12. Geschmacksverstärker, die eine Verbindung der Ansprüche 1 bis 8 in einer Beimischung mit Mononatriumglutamat und/oder mit 5'-Monophosphatnukleotiden umfassen.

13. Nahrungsmittelzubereitungen, die die Zusammensetzungen von Anspruch 12 enthalten.

## Revendications

1. Composés de formule générale (I) 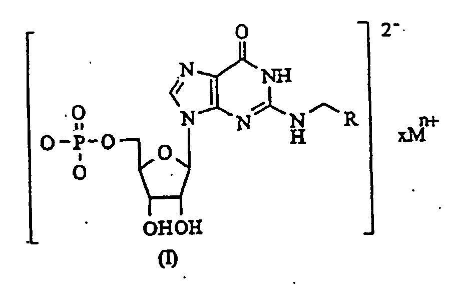 dans laquelle R est :
- alkyle en C₁-C₄, éventuellement substitué par un groupement S-R¹, où R¹ est alkyle en C₁-C₃ ;
- un groupement phényle, benzyle, thiophényle ou benzothiophényle, éventuellement substitué par 1-5 substituants, de préférence de 1 à 3 substituants qui peuvent être identiques ou différents, choisis parmi les groupements -NO₂, -CHO, -R¹ ou -SR¹, où R¹ présente la signification définie ci-dessus ;
- M est hydrogène, un métal alcalin ou alcalino-terreux ;
- X est 1 lorsque n vaut 2 et X est 2 lorsque n vaut 1 ;
à l'exclusion des composés de formule (I) dans laquelle R est méthyle ou propyle non substitué, M est hydrogène, X est 2 et n vaut 1.

2. Composés selon la revendication 1, dans lesquels R est alkyle en C₁-C₃ linéaire, éventuellement substitué par un groupement S-R¹, où R¹ est méthyle, soumis à l'exclusion dans la revendication 1.

3. Composés selon la revendication 1, dans lesquels R est un groupement phényle, thiophényle ou benzothiophényle, éventuellement substitué par un groupement -NO₂, méthyle ou -SR¹, où R¹ est tel que défini dans la revendication 1.

4. Composés selon l'une quelconque des revendications 1 à 3, dans lesquels M est l'hydrogène, le sodium, le potassium, le calcium, le magnésium ou le baryum.

5. Composés selon la revendication 1, dans lesquels M est l'hydrogène, le sodium, le potassium, le calcium, le magnésium ou le baryum et R est :
- alkyle en C₁-C₃ linéaire, éventuellement substitué par un groupement -SCH₃, soumis à l'exclusion dans la revendication 1 ; ou
- un groupement phényle, thiophényle ou benzothiophényle, éventuellement substitué par un groupement -NO₂, méthyle ou -SCH₃.

6. Composés selon l'une quelconque des revendications 1 à 5, dans lesquels M est l'hydrogène ou le sodium.

7. Composé selon la revendication 1, qui est choisi parmi :
le guanosine-5'-monophosphate de N²-(3-méthylthio)propyle ;
le guanosine-5'-monophosphate de N²-(4-méthylthiophényl)méthyle ;
le guanosine-5'-monophosphate de N²-(2-méthylthiophényl)méthyle ;
le guanosine-5'-monophosphate de N²-thiophén-2-ylméthyle ;
le guanosine-5'-monophosphate de N²-thiophén-3-ylméthyle ;
le guanosine-5'-monophosphate de N²-(5-méthylthiophén-2-yl)méthyle ;
le guanosine-5'-monophosphate de N²-(3-méthylthiophén-2-yl)méthyle ;
le guanosine-5'-monophosphate de N²-(3-éthylthiophén-2-yl)méthyle ;
le guanosine-5'-monophosphate de N²-(3-nitrothiophén-2-yl)méthyle ;
le guanosine-5'-monophosphate de N²-(thianaphtén-3-yl)méthyle
et les sels de sodium correspondants.

8. Composé selon la revendication 7, qui est le guanosine-5'-monophosphate de N²-(3-méthylthio)propyle et le sel de sodium correspondant.

9. Utilisation des composés selon l'une quelconque des revendications 1 à 8 en tant qu'exhausteurs de goût dans des préparations alimentaires.

10. Utilisation des composés selon l'une quelconque des revendications 1 à 8, seuls ou en combinaison avec du glutamate monosodique et/ou avec des nucléotides 5'-monophosphate, en tant qu'exhausteurs de goût dans des préparations alimentaires.

11. Utilisation selon la revendication 9 ou 10 pour la préparation de pâtes alimentaires, de risottos, de soupes, de soupes déshydratées ou humides, de snacks, de sauces, d'assaisonnements pour salades, de sauces rouges et blanches prêtes à l'emploi, de bouillons en cubes, de préparations de soupes, de viandes préparées cuites et non cuites, de viandes en boîte, de pâtes alimentaires farcies et de légumes en conserve.

12. Exhausteurs de goût comprenant un composé selon les revendications 1 à 8 en mélange avec du glutamate monosodique et/ou avec des nucléotides 5'-monophosphate.

13. Préparations alimentaires contenant les compositions de la revendication 12.
